# EUROPEAN PATENT SPECIFICATION

(11) **EP 3 843 813 B1**
(45) Date of publication and mention of the grant of the patent: **14.10.2026**
(21) Application number: 19749739.9
(22) Date of filing: 08.08.2019
(51) Int. Cl.: A61M 5/158, A61M 5/32, A61M 5/142, A61M 25/06

(54) **METHOD AND DEVICES FOR RECEIVING A CANNULA**
VERFAHREN UND VORRICHTUNGEN ZUR AUFNAHME EINER KANÜLE
PROCÉDÉ ET DISPOSITIFS POUR RECEVOIR UNE CANULE

(30) Priority: 27.08.2018 EP 18190987
(43) Date of publication of application: 07.07.2021
(73) Proprietor: F. Hoffmann-La Roche AG, 4070 Basel (CH); Roche Diabetes Care GmbH, 68305 Mannheim (DE)
(72) Inventor: DECK, Frank, 68305 Mannheim (DE)
(74) Representative: Pfaffmann, Lucas
(86) International application number: PCT/EP2019/071363
(87) International publication number: WO 2020/043459

(56) References cited:
- WO-A1-2008/014792
- WO-A1-2009/001346
- WO-A1-2015/164647
- US-A1- 2007 191 772

## Description

### Technical Field

The invention relates to a cannula cartridge for receiving a cannula, a kit comprising the cannula cartridge and a method of manufacturing the cannula cartridge. The method and devices according to the present invention may mainly be used when delivering medicine, such as for example insulin, to a user. The invention may both be applied in the field of home care as well as in the field of professional care, such as in hospitals and nursing homes. Other applications are generally feasible.

### Background art

Delivering medicine to a user, specifically insulin delivery, plays an important role in the prevention and treatment of diseases, in particular in the treatment of diabetes mellitus. In medical therapy, when delivering medicine to a user on a regular basis, ambulatory infusion pumps such as medication pumps are widely used. In general, a cannula, such as an infusion cannula, is inserted into a body tissue of a user for the purpose of delivering medicine directly into the user's body, for example via the medication pump. For hygienic and safety reasons such cannulas usually are single use objects and are therefore disposed right after use.

In general, cannulas pose a risk of injury. Therefore, as an example, single use inserter devices are used for insertion of a soft cannula into a body, preventing unintentional contact before and after insertion of the cannula. Such an inserter device may for example be known from EP 1 383 560 B1 disclosing a device for inserting a cannula into tissue, including a cannula, a protective element which can accommodate said cannula, an operating element for moving the cannula out of the protective element, and a holder fixedly connected to the cannula. The invention encompasses a system for connecting a liquid supply to the cannula.

Further, as an alternative, single use cannula cartridges are used for use in reusable inserters are known in the art, specifically for preventing unintentional contact with the cannula. As an example, WO 2009/001346 A1 discloses an insertion apparatus for use with a device for delivery of a therapeutic fluid into a body of a patient and/or for sensing of a bodily analyte. The apparatus includes a housing adapted for loading therein at least one cannula cartridge unit having a protective member. The protective member accommodates at least one penetrating cartridge having a subcutaneously insertable element and a penetrating member. The apparatus includes a displacement mechanism capable of protracting the penetrating cartridge towards the body of the patient, where protraction of the penetrating cartridge results in insertion of the subcutaneously insertable element into the body of the patient.

Despite the advantages involved in using such protection elements, e.g. single use inserter devices and cannula cartridges, several technical challenges remain. Specifically, high insertion forces generally lead to significant inconvenience for users and/or patients. Further, discard rates, so far, in general are related to material induced safety issues. Thus, it is desirable to reduce material failure and fatigue, for example caused by mechanical stress during transport and handling as well as long storage periods.

WO2008/014792 A1 is directed to a device for insertion of a cannula of e.g. an infusion set. The insertion device comprises a first insertion part and a second insertion part, wherein the first insertion part covers an injection needle in a non-activated position. In an activated position the injection needle projects beyond the first insertion part. At least a part of the second insertion part and the first insertion part can be moved relation to each other between at least one activated position and at least one non-activated position, wherein the second insertion part exceeds the distal end of the first insertion part when the device is in an activated position. The first insertion part is provided with a protrusion formed as a cylindrical tap positioned at a part of the exterior surface of the first insertion part which is facing towards an inner surface of the second insertion part. The tap interacts with an opening in the form of a slit in the second insertion part.

WO 2009/001346 A1 is directed to an insertion apparatus for use with a device for delivery of a therapeutic fluid into a body of a patient and/or for sensing of a bodily analyte. The apparatus comprises a housing adapted for loading therein at least one cannula cartridge unit having a protective member. The protective member accommodates at least one penetrating cartridge having a subcutaneously insertable element and a penetrating member. The apparatus further comprises a displacement mechanism capable of protracting the penetrating cartridge towards the body of the patient, where protraction of the penetrating cartridge results in insertion of the subcutaneously insertable element into the body of the patient.

US 2007/191772 A1 is directed to an infusion set assembly comprising a cannula housing having a base surface and a septum housing adapted to be pivotally attached to the cannula housing for movement between a position where an axis thereof is substantially perpendicular to the base surface and a position where the axis is substantially parallel to the base surface. The infusion set assembly further comprises an insertion needle assembly including an insertion needle and a cannula supported by the needle assembly with a tip of the insertion needle extending from a distal end of the cannula. An insertion guide housing is configured to support the insertion needle assembly with the needle substantially perpendicular to the base surface and adapted for movement between a position where the needle tip and cannula distal end are spaced apart from the base surface and a position where the needle tip and cannula distal end extend beyond the base surface.

WO 2015/164647 A1 is directed to an insulin infusion device. The infusion device includes a body, a cannula housed in the body, a cannula insertion device and a cannula retraction device. When the cannula insertion device is activated, the cannula extend at least partly out of the body and into an infusion site and when the cannula retraction device is activated after the cannula insertion device has been activated, the cannula retracts into the body.

### Problem to be solved

It is therefore desirable to provide methods and devices which address the above-mentioned technical challenges of cannula cartridges. Specifically, methods and devices shall be proposed providing a high degree of safety and reliability when handling and inserting the cannula, while still allowing for a compact and easy to use construction.

### Summary

This problem is addressed by a cannula cartridge for receiving a cannula, a kit comprising the cannula cartridge and a method of manufacturing the cannula cartridge with the features of the independent claims. Advantageous embodiments which might be realized in an isolated fashion or in any arbitrary combinations are listed in the dependent claims.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

In a first aspect of the present invention, a cannula cartridge for receiving a cannula is disclosed. The term "cannula cartridge" as used herein is a broad term and is to be given its ordinary and customary meaning to a person of ordinary skill in the art and is not to be limited to a special or customized meaning. The term specifically may refer, without limitation, to an arbitrary protective device configured for housing the cannula.

The cannula cartridge comprises:
a) a needle unit having a needle hub and a needle attached to the needle hub;
b) a cannula, specifically a soft cannula; and
c) a protective element.

The term "needle" as used herein is a broad term and is to be given its ordinary and customary meaning to a person of ordinary skill in the art and is not to be limited to a special or customized meaning. The term specifically may refer, without limitation, to an arbitrary elongated object having a sharp tip. In particular, the needle may have an elongated shape having a circular or oval profile and wherein a length of a longitudinal extension of the needle exceeds a diameter of the profile by at least 10 times. For example the profile may change over the length of the needle. The needle may be or may comprise at least one solid and/or rigid material, such as a metal material, for example steel. The needle may specifically comprise two ends or edges, wherein one end may be attached to the needle hub and the other end may comprise the sharp tip. The needle, specifically with the sharp tip, may for example be configured for piercing or puncturing a skin of the user. Specifically, the sharp tip may be slightly beveled, in particular the sharp tip may have at least one angled or chamfered side.

The term "needle hub" as used herein may specifically refer, without limitation, to an arbitrary connecting element attached or attachable to a needle. The needle hub may specifically be or may comprise means for applying force to the needle. The needle hub may at least partially have a cylindrical shape. Specifically, the needle hub may be of or may comprise at least one plastic material or polymer, such as a thermoplastic material or a thermosetting material, for example acrylonitrile butadiene styrene (ABS) or polypropylene (PP). In particular, propylene may for example be used for being at least partially transparent, specifically for ultraviolet (UV) radiation or UV light, e.g. electromagnetic radiation having wavelengths in the range of 10 nm to 100 nm. Thus, in particular, the material of the needle hub may for example allow electromagnetic radiation, e.g. UV light, to pass through the material. For example, the passing of electromagnetic radiation may be beneficial for curing or hardening of UV curing adhesives, such as an adhesive generating a crosslinked network of for example polymers, when irradiated by UV light.

The term "cannula" as used herein is a broad term and is to be given its ordinary and customary meaning to a person of ordinary skill in the art and is not to be limited to a special or customized meaning. The term specifically may refer, without limitation, to an arbitrary fluidic element for conducting a liquid, which may be or may comprise a hollow tube or a hollow needle. The cannula may comprise a round profile, for example the profile may have the shape of an O. Other shapes of the profile are also feasible. For example the profile may change over the length of the cannula. The cannula may have two opposing ends. The cannula may particularly be configured to establish a fluid connection between the two opposing ends of the cannula. Specifically, the hollow profile of the cannula may complement the profile of the needle, such that the needle may be an exact fit into or through the cannula. In particular, the cannula may be slid on the needle.

The needle unit is movable within the protective element from at least one first position to at least one second position and vice a versa. In the first position the needle is covered by the protective element and in the second position the needle at least partially extends from the protective element.

The term "protective element" as used herein is a broad term and is to be given its ordinary and customary meaning to a person of ordinary skill in the art and is not to be limited to a special or customized meaning. The term specifically may refer, without limitation, to an arbitrary object having a protection space and configured to cover or shield an arbitrary element held within the protected space. Specifically, in the first position the needle unit may be held within the protected space of the protective element and thus, in the first position the needle unit may be covered by the protective element. The protective element may specifically be made of or may comprise at least one plastic material or polymer, such as a thermoplastic material or a thermosetting material, for example acrylonitrile butadiene styrene (ABS) or polypropylene (PP). In particular, the protective element may specifically be made of or may comprise a material having a friction coefficient µ of 0.005 ≤ µ ≤ 0.5, specifically 0.01 ≤ µ ≤ 0.45, more specifically 0.02 ≤ µ ≤ 0.3.

Additionally, in the first position a retaining member of the protective element engages with an engagement element of the needle hub by a form fit connection. The term "form fit connection" as used herein is a broad term and is to be given its ordinary and customary meaning to a person of ordinary skill in the art and is not to be limited to a special or customized meaning. The term specifically may refer, without limitation, to an assembly of at least two objects via at least partially conforming shapes or outlines. Thus, the retaining member and the engagement element may each comprise at least one element, both having essentially complementary forms or shapes in order to engage by the form fit connection. As an example, the retaining member may show a protrusion or convexity where the engagement element may show an indentation or concavity, thus in order for the retaining member and the engagement element to connect by a form fit, the protrusion and the indentation may engage.

The needle unit is configured to be moved by an inserter from the first position to the second position and back to the first position in which the retaining member again engages with the engagement element of the needle hub. The term "inserter" as used herein is a broad term and is to be given its ordinary and customary meaning to a person of ordinary skill in the art and is not to be limited to a special or customized meaning. The term specifically may refer, without limitation, to an arbitrary system configured for inserting at least one object into an arbitrary element by guiding a movement of the at least one object. In particular, the inserter may be configured for inserting the cannula into a body tissue of a user. As an example, the cannula may be slid onto the needle, e.g. during an assembly of the cannula cartridge, and may be inserted into the body tissue of the user via the movement of the needle unit guided by the inserter.

The retaining member and the engagement element of the needle hub are configured such that a holding force of the retaining member is overcome by applying forces parallel to the axis of the needle.

Further, the retaining member may comprise a spring element. In particular, the spring element may for example be configured for elastically return to a starting or initial position after being deflected. The spring element specifically may contain an interlocking element configured to snap into a corresponding feature of the engagement element. Specifically, the interlocking element may be configured to form a form fit connection with the engagement element.

The engagement element may comprise at least one groove or indentation in the needle hub. In particular, the at least one groove of the engagement element may for example be a circumferential groove or narrow channel. Specifically, the circumferential groove or narrow channel, for example an all-around indentation, of the needle hub may divide the engagement element into at least two parts, such as at least two halves. In particular, the needle hub may have an essentially cylindrical shape, with the circumferential groove dividing the needle hub into two halves, specifically for example forming the shape of a barbell or an hourglass shape.

The retaining member contains at least one latch. In particular, the latch, for example a protrusion, may be configured for engaging with at least one cavity of the engagement element, such as with the at least one indentation or groove of the engagement element. Specifically, the latch may be configured for at least partially engaging with the at least one cavity, for example with the at least one groove in the outer surface of the engagement element, e.g. with the circumferential groove. Thus, as an example the latch of the retaining member may for example be configured for at least partially engaging with the circumferential groove of the engagement element.

In an engaged state, the latch may block a motion of the needle unit for example both in a first direction pointing from the first position to the second position and in a second direction being opposite to the first direction. In particular, in the engaged state, the latch may block a movement of the needle along an axis of the needle. Specifically a movement in both directions along the axis of the needle may be blocked.

The latch may be configured such that the blocking of the motion in the first direction may be overcome by a first biasing force on the needle unit. The latch may further be configured such that the blocking of the motion in the second direction may be overcome by a second biasing force on the needle unit.

Further, the latch comprises at least one latch arm. The latch arm is specifically connected to a housing of the protective element at a fixed end. In addition, the latch arm further has at least one free end configured to engage with the engagement element of the needle hub.

The free end of the latch arm may for example contain an interlocking element having at least two sloped surfaces configured for engaging the engagement element of the needle hub. In particular, the at least two sloped surfaces may have different slopes, specifically different absolute values of the slopes. As an example, the at least two sloped surfaces may comprise at least one first sloped surface interacting with at least one edge of the engagement element when the needle unit moves from the first position to the second position, and at least one second sloped surface interacting with at least one proximal edge of the needle hub when the needle unit moves back from the second position to the first position.

The free end of the latch arm contains an interlocking element configured for engaging the engagement element of the needle hub, wherein the interlocking element and the engagement element of the needle hub together comprise at least two sloped surfaces. The at least two sloped surfaces comprise at least one first sloped surface slidably engaging the engagement element with the interlocking element when the needle unit moves from the first position to the second position, and at least one second sloped surface slidably engaging the engagement element with interlocking element when the needle unit moves back from the second position to the first position, such that by moving the needle unit from the first position to the second position the form fit connection is unlocked and by moving the needle unit from the second position to the first position the form fit connection is locked. The first sloped surface may be part of the engagement element or the interlocking element, respectively. Likewise, the second sloped surface may be part of the engagement element or the interlocking element, respectively. Each sloped surface interacts with a respective edge or surface of the respective other element so to form a form fit connection. For example, in an embodiment the interlocking element comprises the at least two sloped surfaces, the at least one first sloped surface interacting with at least one edge of the engagement element when the needle unit moves from the first position to the second position, and the at least one second sloped surface interacting with at least one proximal edge of the needle hub, when the needle unit moves back from the second position to the first position, such that by moving the needle unit from the first position to the second position the form fit connection is unlocked and by moving the needle unit from the second position to the first position the form fit connection is locked.

Further, the interlocking element may contain a protrusion from the latch arm, wherein the sloped surfaces may for example be surfaces of the protrusion and may specifically be configured for interacting with different edges of the needle hub.

The latch arm may specifically extend in a direction parallel to the axis of the needle. Thus, a longitudinal side of the latch arm may for example be arranged parallel to a longitudinal extension direction of the needle, specifically parallel to the axis of the needle. The latch arm may for example be disposed in a slot within a housing of the protective element. In particular, the latch arm may be disposed in a slot extending up to a rim of the housing at a proximal end of the housing.

In particular, the latch, when the retaining member is engaged with the engagement element of the needle hub, may be in a relaxed state. Thus, in an engaged state, when the retaining member is engaged with the engagement element of the needle hub, the latch may specifically be in a non-deflected state. As an example, in the relaxed state, specifically in the non-deflected state, no tensions may occur within the latch due to deformation. Thus, in the relaxed state, no deformation of the latch may occur. In particular, the latch may be free of inner stress or strain.

A retaining force of the retaining element may for example be defined by an elasticity of the latch and at least one angle of the latch. In particular, the form or shape as well as the material properties of the latch may dictate or define the retaining force of the retaining element.

The protective element may specifically be a monolithic element. In particular, the monolithic element may for example be an injection molded piece. The protective element, specifically in case of being injection molded, may be at least partially made of a thermoplastic material, such as for example polypropylene or ABS.

The protective element may for example comprise at least one longitudinal slot extending parallel to the axis of the needle. Further, the protective element may for example comprise at least one handling tab extending from the housing of the protective element. In particular, the handling tab may be configured for providing a surface where a user may hold the protective element, specifically using his or her fingers. Further, the protective element may, at its distal end, comprise at least one receptacle for receiving a centering element on a pump holder.

The protective element may have a housing with a bore contained therein. In particular, the needle unit may be received within the bore in a linearly slidable fashion in a direction parallel to the axis of the needle. The bore may specifically be a cylindrical bore.

In the first position, a proximal rim of the needle hub may be essentially flush with an upper rim of the protective element. In particular, in the first position, the proximal rim of the needle hub, specifically the upper rim of the needle hub, may be arranged in the same plane as the upper rim of the protective element. In the second position a lower rim of the needle hub may be essentially flush with a distal rim of the protective element. Specifically, in the second position the lower rim of the needle hub, e.g. a distal rim of the needle hub, may be arranged in the same plane as the distal rim of the protective element.

Specifically, in the second position, the needle and the cannula may protrude or extend from the protective element such that the cannula may be at least partially inserted into an adjoining element or object, such as for example a body tissue of a user or patient. Thus, in the second position, a gap may exist between the lower rim of the needle hub and the distal rim of the protective element. As an example, the lower rim of the needle hub may for example be essentially flush with a surface of the adjoining element or object, in particular with a surface of the body tissue.

The protective element may further contain at least one securing element for preventing the needle unit from leaving the protective element in a direction from the second position to the first position. In particular, the protective element may contain at least one protrusion protruding from a proximal rim of a bore within the protective element, the bore receiving the needle unit.

The cannula may specifically comprise a soft tubular cannula received on the needle. Specifically, the cannula may be or may comprise at least one flexible material, for example a flexible polymer material. In particular, the soft cannula may be received or slid onto the needle, in order to stabilize the soft cannula, specifically when inserting the cannula into the body tissue. The cannula may further comprise at least one cannula hub attached to the soft tubular cannula.

In a further aspect of the present invention, a kit is disclosed. The kit comprises:
A) at least one cannula cartridge; and
B) at least one inserter for inserting the cannula into a body tissue.

In particular, the at least one cannula cartridge may specifically be the cannula cartridge as indicated above or as further described below. Thus, for possible additional definitions and embodiments reference may be made to the description given above.

The inserter comprises:
- at least one inserter housing having at least one receptacle for receiving the cannula cartridge; and
- at least one insertion actuator configured for driving the needle unit from the first position to the second position and back to the first position.

The insertion actuator may be spring-driven. Thus, the insertion actuator may rely on a spring-driven mechanism for driving the needle unit from the first position to the second position and back to the first position. In particular, the insertion actuator may be triggered by a spring.

Further, the insertion actuator may comprise at least one driving element interlocking with the engagement element of the needle hub during driving the needle unit. The driving element may specifically contain a fork clasping the needle hub from at least two sides during driving the needle unit.

In particular, the engagement element of the needle hub may contain a circumferential groove, wherein the fork interlocks with the circumferential groove. Specifically, fork and retaining member may interact with the engagement element of the needle hub from opposing sides.

Additionally or alternatively however, the engagement element of the needle hub may comprise at least two indentations, such as separate indentations, for example two symmetrically arranged indentations, wherein the fork may interlock with the at least two indentations. Thus, for example, the engagement element of the needle hub may, specifically additionally or alternatively to the at least one circumferential groove, comprise multiple indentations, wherein the form may interact with the engagement element of the needle hub via the at least two indentations from one side and wherein the retaining member may interact with the engagement element of the needle hub from an opposing side, for example via at least one further indentation or groove in the needle hub.

The inserter may further comprise at least one actuating element, specifically a trigger, the actuating element being configured for being actuated by a user in order to initiate the insertion of the cannula into the body tissue.

The kit may further comprise:
C) at least one pump holder comprising:
- at least one attachment surface for attachment to a body surface of the user, specifically having at least one adhesive element; and
- at least one mounting surface opposing the attachment surface, the mounting surface having at least one mounting element for mounting either the inserter or a medication pump, specifically an insulin pump, to the pump holder.

Further, the pump holder may comprise a base providing the attachment surface and the mounting surface on opposing sides. In particular, the base may have at least one opening disposed therein for providing passage for one or more of the needle or the cannula. Further, the kit may comprise:
D) at least one medication pump mountable onto the pump holder, wherein the medication pump and the inserter may be interchangeable.

In a further aspect of the present invention, a method of manufacturing the cannula cartridge is disclosed. In particular, the cannula cartridge as indicated above or as further described below is manufactured by the method. The method comprises the steps disclosed in the following. The steps may specifically be performed in the given order. Still, a different order is possible. The method may comprise additional steps which are not mentioned. It is further possible to perform one or more or all of the method steps repeatedly. Further, two or more of the method steps may be performed simultaneously or in a timely overlapping fashion.

The method comprises the following steps:
i. providing the needle unit;
ii. providing the cannula;
iii. providing the protective element;
iv. mounting the cannula to the needle of the needle unit;
v. inserting the needle unit with the cannula disposed thereon into the protective element, in a direction such that the needle of the needle unit is inserted ahead.

The devices and methods according to the present invention may provide a large number of advantages over known methods and devices. In particular, the disclosed cannula cartridge and the kit may further improve the security of handling and using the devices. Specifically, an impact of manufacturing tolerances and temperature effects on the safety of handling and using the disclosed devices may be reduced compared to known methods and devices. Thus, in particular, discard rates due to material related safety issues may be minimized, and manufacturing yield may be increased. For example, in the proposed devices and methods, material failure and fatigue caused by mechanical stress during transport and handling as wells as long storage periods, may be significantly reduced compared to known methods and devices.

Further, the devices and methods suggested in the present invention may improve a production process of the proposed devices. In particular, the production of the proposed devices may be more economic due to a decrease of discard rates compared to methods and devices known from the art. Furthermore, a decrease of the discard rates may also lead to a more efficient use of resources. Thus, the devices and methods according to the present invention may further show a higher degree of environmental compatibility than known methods and devices.

Further, the reliability of the devices and methods may be increased. Thus, as an example, the use of the engagement element and the retaining member may be used for replacing force-fit connections such as press-fit or interference-fit connections for holding the needle unit in place. Press-fit or interference-fit connections are prone to be subject to manufacturing tolerance issues and, thus, may create reliability issues. By using the engagement element and the retaining member, as described above, these issues may be avoided, and precision and reproducibility of the insertion process may be improved.

In addition, the proposed methods and devices may significantly lessen a user's or patient's inconvenience compared to known methods and devices, when inserting the cannula into the body tissue of the user. In particular, the cannula cartridge and the kit as disclosed herein may for example allow an insertion of the cannula with lower insertion forces than devices known in the art.

### Short description of the Figures

Further optional features and embodiments will be disclosed in more detail in the subsequent description of embodiments, preferably in conjunction with the dependent claims. Therein, the respective optional features may be realized in an isolated fashion as well as in any arbitrary feasible combination, as the skilled person will realize. The scope of the invention is not restricted by the preferred embodiments. The embodiments are schematically depicted in the Figures. Therein, identical reference numbers in these Figures refer to identical or functionally comparable elements.

In the Figures:
- Figure 1: shows a perspective view of an embodiment of a kit comprising an embodiment of a cannula cartridge;
- Figure 2: shows an embodiment of a cannula cartridge in a perspective view;
- Figures 3A and 3B: show section views of an embodiment of a cannula cartridge with the needle unit and the cannula in a first position (Figure 3A) and in a second position (Figure 3B);
- Figure 4: shows a detail of an embodiment of a cannula cartridge; and
- Figure 5: shows a flow chart of an embodiment of a method of manufacturing a cannula cartridge.

### Detailed description of the embodiments

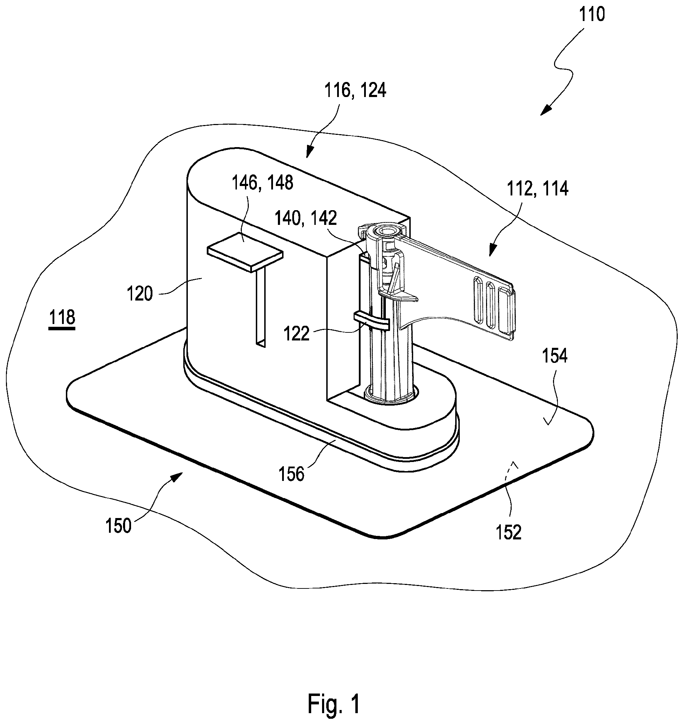

Figure 1 shows a perspective view of an embodiment of a kit 110 comprising at least one cannula cartridge 112 for receiving a cannula 114. The kit 110 further comprises at least one inserter 116 for inserting the cannula 114 into a body tissue 118. The inserter 116 comprises at least one inserter housing 120 having at least one receptacle 122 for receiving the cannula cartridge 112 and at least one insertion actuator 124.

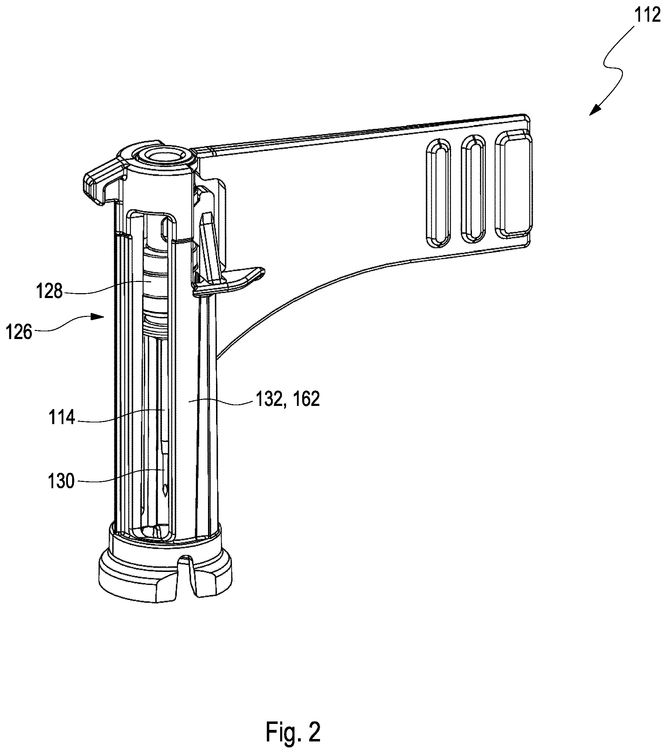

Figure 2 shows an embodiment of the cannula cartridge 112 comprising the cannula 114 and a needle unit 126 having a needle hub 128 and a needle 130 attached to the needle hub 128. In particular, the cannula 114, for example a soft cannula 114, may be mounted to the needle 130 of the needle unit 126. Specifically, the cannula 114 may be slid onto the needle 130. The cannula cartridge 112 further comprises a protective element 132.

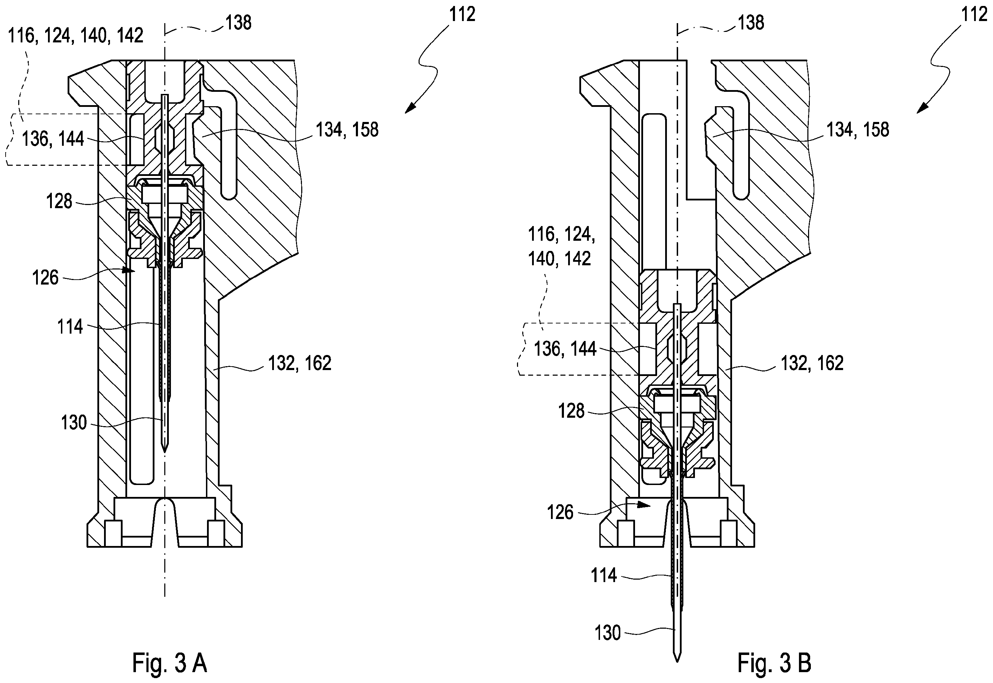

In particular, the needle unit 126 is movable within the protective element from at least one first position, as for example illustrated in Figure 3A, to at least one second position, as for example illustrated in Figure 3B, and vice versa. As shown in Figure 3A, in the first position the needle 130 is covered by the protective element 132. As shown in Figure 3B, in the second position the needle 130 at least partially extends from the protective element 132. In the first position a retaining member 134 of the protective element 132 engages with an engagement element 136 of the needle hub 128 by a form fit connection.

The needle unit 126 is configured to be moved by an inserter 116 from the first position to the second position and back to a the first position in which the retaining member 134 again engages with the engagement element 136 of the needle hub 128. Further, the retaining member 134 and the engagement element 136 of the needle hub 128 are configured such that a holding force of the retaining member 134 is overcome by applying forces parallel to the axis 138 of the needle 130.

In particular, the insertion actuator 124 as illustrated in Figure 1 is configured for driving the needle unit 126 from the first position to the second position and back to the first position. The insertion actuator 124 may for example be spring driven and may for example comprise at least one driving element 140 interlocking with the engagement element 136 of the needle hub 128 during driving the needle unit 128. Specifically, as illustrated in Figures 3A and 3B, the driving element 140 may comprise a fork 142 clasping the needle hub 128 from at least two sides during driving the needle unit 126. In particular, the needle hub 128 may comprise at least one circumferential groove 144, wherein the fork 142 may interlock with the circumferential groove 144. As illustrated in the figures, the fork 142 and the retaining member 134 may interact with the engagement element 136 of the needle hub 128 from opposing sides.

In particular, Figure 3B, may specifically illustrate an optional embodiment of the cannula cartridge 112 with the needle unit 126 and the cannula 114 in the second position. Specifically the positioning of the needle unit 126 and the cannula 114 in the second position may differ from the illustrated arrangement. For example, the needle unit 126 and the cannula 114 may, in the second position, be arranged such that a lower rim of the needle hub 128 may be essentially flush to either a distal rim of the protective element 132 or a surface of the body tissue 118. However, other arrangements are feasible. Specifically, in the second position, the needle 130 and the cannula 114 may protrude or extend from the protective element 132 such that the cannula 114 may be at least partially inserted into the body tissue 118. Thus, as an example, in the second position, a gap may exist between the lower rim of the needle hub 128 and the distal rim of the protective element 132.

As illustrated in Figure 1, the inserter 116 may further comprise at least one actuating element 146, specifically a trigger 148. The actuating element 146 may for example be configured for being actuated in order to initiate the insertion of the cannula 114 into the body tissue 118. Specifically, the actuating element 146 may be configured for being activated by a user. Alternatively however, the actuation element 146 may for example be configured for being activated by a press-force, such as a force occurring when positioning the inserter on top of the body tissue 118. Thus, as an example, the press-force may activate the actuation element 146 in the event of the inserter 116 being positioned relative to the body tissue 118. As further illustrated in Figure 1, the kit 110 may comprise at least one pump holder 150. In particular, the pump holder 150 may comprise at least one attachment surface 152 for attachment to a body surface of the user, such as to a surface of the body tissue 118 of the user. The attachment surface 152 may for example have at least one adhesive element. The pump holder 150 may further comprise at least one mounting surface 154 opposing the attachment surface 152. Specifically, the mounting surface 154 may have at least one mounting element 156 for mounting either the inserter 116 or a medication pump (not shown), specifically an insulin pump, to the pump holder 150.

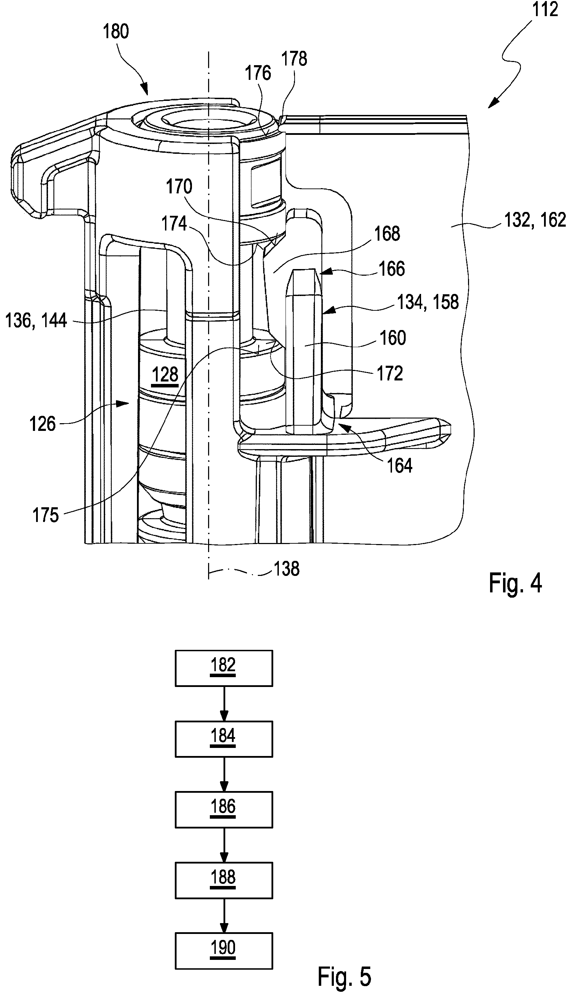

Figure 4 shows an embodiment of a detail of an embodiment of a cannula cartridge, specifically a detail of the embodiment of the cannula cartridge as illustrated in Figure 2. As illustrated, the needle hub 128 may have a cylindrical shape, wherein as an example, the engagement element 136 of the needle hub 128, specifically the circumferential groove 144 of the engagement element 136, may divide the needle hub 128 into two halves. Thus, the circumferential groove 144 of the engagement element 136 may for example form the needle hub 128 into the shape of a barbell. In Figure 4, the needle unit 126 is illustrated in the first position, thus the engagement element 136 of the needle hub 128 in Figure 2 is engaged with the retaining member 134 of the protective element 132 by a form fit connection. As illustrated, the retaining member 134 may comprise at least one latch 158, wherein the latch 158 may be configured to engage with at least one cavity in at least one surface of the engagement element 136, such as with the circumferential groove 144. In particular, the latch 158 may comprise at least one latch arm 160, for example extending in a direction parallel to the axis 138 of the needle 130. The latch arm 160 may specifically be connected to a housing 162 of the protective element 132 at a fixed end 164. Further, the latch arm 160 may have at least one free end 166 configured to engage with the engagement element 136 of the needle hub 128. As an example, the free end 166 may contain an interlocking element 168 having at least a first sloped surface 170 and a second sloped surface 172 for engaging the engagement element 136. The at least two sloped surfaces 170, 172 may specifically have different slopes, in particular different absolute values of the slopes. For example, the first sloped surface 170 may interact with at least one edge 174 of the engagement element 136 when the needle unit 126 moves from the first position to the second position. The second sloped surface 172 on the other hand, may interact with at least one proximal edge 176 of the needle hub 126 when the needle unit 126 moves back from the second position to the first position. Further, the second sloped surface 172 may additionally interact with at least one lower edge 175 of the engagement element 136, specifically as an example, in order to keep the needle unit 126 from leaving the protective element 132. In particular, the latch arm 160 may be disposed in a slot within the housing 162 of the protective element 132, wherein the slot may extend up to a rim 178 of the housing 162 at a proximal end 180 of the housing 162. The latch arm 160 may specifically be in a relaxed state, for example in a non-deflected state, when the retaining member 134 is engaged with the engagement element 136 of the needle hub 128.

As an example, in the first position, the latch 158 may keep the needle hub 128, specifically the needle unit 126 having the cannula 114 mounted thereon, in place, in particular by the interlocking element 168 with its two sloped surfaces 170, 172 for engaging the engagement element 136. The latch 158 itself may, specifically in the first position, be in a relaxed or tension-free state. Thus, no material deformation due to stress and/or strain may occur in the latch 158 in the first position. A retaining force of the retaining member 134, specifically of a retaining element or the latch 158, may be defined by an elasticity of the latch 158 and at least one angle of the latch, in particular by the first sloped surface 170 and/or the second sloped surface 172. Further, the retaining force may depend on a material combination, specifically on at least one friction coefficient, of the latch 158 and the needle hub 128. A movement or shift of the needle unit 126 from the first position in the direction of the second position along the axis 138 of the needle 130 may specifically require overpowering the retaining force. In case the retaining force is overpowered, specifically the latch 158 may be deflected such that the needle unit 126 may be slidable from the first position in the direction towards the second position. The latch 158 may return to a non-deflected state after the needle hub 128 has for example passed the latch 158.

Figure 5 shows a flow chart of an embodiment of a method of manufacturing a cannula cartridge 112. The method comprises the following steps:
i. (denoted with reference number 182) providing the needle unit 126;
ii. (denoted with reference number 184) providing the cannula 114;
iii. (denoted with reference number 186) providing the protective element 132;
iv. (denoted with reference number 188) mounting the cannula 114 to the needle 130 of the needle unit 126;
v. (denoted with reference number 190) inserting the needle unit 126 with the cannula 114 disposed thereon into the protective element 132, in a direction such that the needle 130 of the needle unit 126 is inserted ahead.

### List of reference numbers

- 110: kit
- 112: cannula cartridge
- 114: cannula
- 116: inserter
- 118: body tissue
- 120: inserter housing
- 122: receptacle
- 124: insertion actuator
- 126: needle unit
- 128: needle hub
- 130: needle
- 132: protective element
- 134: retaining member
- 136: engagement element
- 138: axis
- 140: driving element
- 142: fork
- 144: circumferential groove
- 146: actuating element
- 148: trigger
- 150: pump holder
- 152: attachment surface
- 154: mounting surface
- 156: mounting element
- 158: latch
- 160: latch arm
- 162: housing
- 164: fixed end
- 166: free end
- 168: interlocking element
- 170: first sloped surface
- 172: second sloped surface
- 174: edge
- 175: lower edge
- 176: proximal edge
- 178: rim
- 180: proximal end
- 182: step i.: providing the needle unit
- 184: step ii.: providing the cannula
- 186: step iii.: providing the protective element
- 188: step iv.: mounting the cannula to the needle of the needle unit
- 190: step v.: inserting the needle unit with the cannula disposed thereon into the protective element, in a direction such that the needle of the needle unit is inserted ahead

## Claims

1. A cannula cartridge (112) for receiving a cannula (114), comprising:
a) a needle unit (126) having a needle hub (128) and a needle (130) attached to the needle hub (128);
b) a cannula (114); and
c) a protective element (132),
- wherein the needle unit (126) is movable within the protective element (132) in a direction parallel to the longitudinal axis of the needle from at least one first position to at least one second position and vice a versa,
- wherein in the first position the needle (130) is covered by the protective element (132),
- wherein in the second position the needle (130) at least partially extends from the protective element (132),
- wherein in the first position a retaining member (134) of the protective element (132) engages with an engagement element (136) of the needle hub (128) by a form fit connection,
- wherein the needle unit (126) is configured to be moved by an inserter (116) from the first position to the second position and back to a the first position in which the retaining member (134) again engages with the engagement element (136) of the needle hub (128),
**characterized in that**
- the retaining member (134) contains at least one latch (158),
- the latch (158) comprises at least one latch arm (160), the latch arm (160) being connected to a housing (162) of the protective element (132) at a fixed end (164), the latch arm (160) further having at least one free end (166) configured to engage with the engagement element (136) of the needle hub (128),
- wherein the free end (166) contains an interlocking element (168) configured for engaging the engagement element (136) of the needle hub (128),
- wherein the interlocking element (168) and the engagement element of the needle hub (128) together comprise at least two sloped surfaces (170, 172), the at least two sloped surfaces (170, 172) comprise at least one first sloped surface (170) slidably engaging the engagement element (136) with the interlocking element (168) when the needle unit (126) moves from the first position to the second position, and at least one second sloped surface (172) slidably engaging the engagement element (136) with the interlocking element (168) when the needle unit (126) moves back from the second position to the first position, such that by moving the needle unit (126) from the first position to the second position the form fit connection is unlocked and by moving the needle unit (126) from the second position to the first position the form fit connection is locked.

2. The cannula cartridge (112) according to the preceding claim, wherein the interlocking element (168) comprises the at least two sloped surfaces (170, 172), the at least one first sloped surface (170) interacting with at least one edge (174) of the engagement element (136) when the needle unit (126) moves from the first position to the second position, and the at least one second sloped surface (172) interacting with at least one proximal edge (176) of the needle hub, when the needle unit moves back from the second position to the first position, such that by moving the needle unit (126) from the first position to the second position the form fit connection is unlocked and by moving the needle unit (126) from the second position to the first position the form fit connection is locked.

3. The cannula cartridge (112) according to any one of the preceding claims, wherein the engagement element (136) comprises a circumferential groove (144) in the needle hub (128).

4. The cannula cartridge (112) according to any one of the preceding claims, wherein the latch (158) is configured to engage with at least one cavity in at least one surface of the engagement element (136).

5. The cannula cartridge (112) according to any one of the preceding claims, wherein the at least two sloped surfaces (170, 172) have different slopes.

6. The cannula cartridge (112) according to any one of the preceding claims, wherein the latch (158), when the retaining member (134) is engaged with the engagement element (136) of the needle hub (128), is in a relaxed state.

7. The cannula cartridge (112) according to any one of the preceding claims, wherein the protective element (132) has a housing (162) with a bore contained therein, wherein the needle unit (126) is received within the bore in a linearly slidable fashion in a direction parallel to the axis (138) of the needle (130).

8. The cannula cartridge (112) according to any one of the preceding claims, wherein the protective element (132) contains at least one securing element for preventing the needle unit (126) from leaving the protective element (132) in a direction from the second position to the first position.

9. A kit (110) comprising:
A) at least one cannula cartridge (112) according to any one of the preceding claims; and
B) at least one inserter (116) for inserting the cannula (114) into a body tissue (118), the inserter (116) comprising:
- at least one inserter housing (120) having at least one receptacle (122) for receiving the cannula cartridge (112); and
- at least one insertion actuator (124) configured for driving the needle unit (126) from the first position to the second position and back to the first position.

10. The kit (110) according to the preceding claim, wherein the insertion actuator (124) comprises at least one driving element (140) interlocking with the engagement element (136) of the needle hub (128) during driving the needle unit (126), wherein the driving element (140) contains a fork (142) clasping the needle hub (128) from at least two sides during driving the needle unit (126).

11. The kit (110) according to any one of the preceding claims referring to a kit (110), wherein the kit (110) further comprises:
C) at least one pump holder (150) comprising:
- at least one attachment surface (152) for attachment to a body surface of the user; and
- at least one mounting surface (154) opposing the attachment surface (152), the mounting surface (154) having at least one mounting element (156) for mounting either the inserter (116) or a medication pump to the pump holder (150).

12. The kit (110) according to the preceding claim, the kit (110) further comprising:
D) at least one medication pump mountable onto the pump holder (150), wherein the medication pump and the inserter (116) are interchangeable.

13. A method of manufacturing the cannula cartridge (112) according to any one of the preceding claims referring to a cannula cartridge (112), the method comprising:
i. providing the needle unit (126);
ii. providing the cannula (114);
iii. providing the protective element (132);
iv. mounting the cannula (114) to the needle (130) of the needle unit (126);
v. inserting the needle unit (126) with the cannula (114) disposed thereon into the protective element (132), in a direction such that the needle (130) of the needle unit (126) is inserted ahead.

## Patentansprüche

1. Kanülenkartusche (112) zum Aufnehmen einer Kanüle (114), umfassend:
a) eine Nadeleinheit (126) mit einem Nadelansatz (128) und einer an dem Nadelansatz (128) angebrachten Nadel (130);
b) eine Kanüle (114); und
c) ein Schutzelement (132),
- wobei die Nadeleinheit (126) innerhalb des Schutzelements (132) in einer Richtung parallel zur Längsachse der Nadel von mindestens einer ersten Position in mindestens eine zweite Position und umgekehrt bewegbar ist,
- wobei in der ersten Position die Nadel (130) von dem Schutzelement (132) bedeckt ist,
- wobei in der zweiten Position die Nadel (130) sich zumindest teilweise von dem Schutzelement (132) erstreckt,
- wobei in der ersten Position ein Halteelement (134) des Schutzelements (132) mit einem Eingriffselement (136) des Nadelansatzes (128) durch eine formschlüssige Verbindung in Eingriff steht,
- wobei die Nadeleinheit (126) konfiguriert ist, um von einer Einführvorrichtung (116) von der ersten Position in die zweite Position und zurück in eine erste Position bewegt zu werden, in der das Halteelement (134) wieder mit dem Eingriffselement (136) des Nadelansatzes (128) in Eingriff steht,
**dadurch gekennzeichnet, dass**
- das Halteelement (134) mindestens einen Riegel (158) enthält,
- der Riegel (158) mindestens einen Riegelarm (160) umfasst, wobei der Riegelarm (160) an einem festen Ende (164) mit einem Gehäuse (162) des Schutzelements (132) verbunden ist, wobei der Riegelarm (160) ferner mindestens ein freies Ende (166) aufweist, das konfiguriert ist, um mit dem Eingriffselement (136) des Nadelansatzes (128) in Eingriff zu kommen,
- wobei das freie Ende (166) ein Verriegelungselement (168) enthält, das konfiguriert ist, um mit dem Eingriffselement (136) des Nadelansatzes (128) in Eingriff zu kommen,
- wobei das Verriegelungselement (168) und das Eingriffselement des Nadelansatzes (128) zusammen mindestens zwei geneigte Flächen (170, 172) umfassen, wobei die mindestens zwei geneigten Flächen (170, 172) mindestens eine erste geneigte Fläche (170), die das Eingriffselement (136) gleitend mit dem Verriegelungselement (168) in Eingriff bringt, wenn sich die Nadeleinheit (126) von der ersten Position in die zweite Position bewegt, und mindestens eine zweite geneigte Fläche (172), die das Eingriffselement (136) gleitend mit dem Verriegelungselement (168) in Eingriff bringt, wenn sich die Nadeleinheit (126) von der zweiten Position zurück in die erste Position bewegt, umfassen, sodass durch Bewegen der Nadeleinheit (126) von der ersten Position in die zweite Position die formschlüssige Verbindung gelöst wird und durch Bewegen der Nadeleinheit (126) von der zweiten Position in die erste Position die formschlüssige Verbindung hergestellt wird.

2. Kanülenkartusche (112) nach dem vorhergehenden Anspruch, wobei das Verriegelungselement (168) die mindestens zwei geneigten Flächen (170, 172) umfasst, wobei die mindestens eine erste geneigte Fläche (170) mit mindestens einer Kante (174) des Eingriffselements (136) zusammenwirkt, wenn sich die Nadeleinheit (126) von der ersten Position in die zweite Position bewegt, und die mindestens eine zweite geneigte Fläche (172) mit mindestens einer proximalen Kante (176) des Nadelansatzes zusammenwirkt, wenn sich die Nadeleinheit von der zweiten Position zurück in die erste Position bewegt, sodass durch Bewegen der Nadeleinheit (126) von der ersten Position in die zweite Position die formschlüssige Verbindung gelöst wird und durch Bewegen der Nadeleinheit (126) von der zweiten Position in die erste Position die formschlüssige Verbindung hergestellt wird.

3. Kanülenkartusche (112) nach einem der vorhergehenden Ansprüche, wobei das Eingriffselement (136) eine Umfangsnut (144) in dem Nadelansatz (128) umfasst.

4. Kanülenkartusche (112) nach einem der vorhergehenden Ansprüche, wobei der Riegel (158) konfiguriert ist, um mit mindestens einem Hohlraum in mindestens einer Fläche des Eingriffselements (136) in Eingriff zu kommen.

5. Kanülenkartusche (112) nach einem der vorhergehenden Ansprüche, wobei die mindestens zwei geneigten Flächen (170, 172) unterschiedliche Neigungen aufweisen.

6. Kanülenkartusche (112) nach einem der vorhergehenden Ansprüche, wobei sich der Riegel (158) in einem entspannten Zustand befindet, wenn das Halteelement (134) mit dem Eingriffselement (136) des Nadelansatzes (128) in Eingriff steht.

7. Kanülenkartusche (112) nach einem der vorhergehenden Ansprüche, wobei das Schutzelement (132) ein Gehäuse (162) mit einer darin enthaltenen Bohrung aufweist, wobei die Nadeleinheit (126) in der Bohrung in einer linear verschiebbaren Weise in einer Richtung parallel zur Achse (138) der Nadel (130) aufgenommen ist.

8. Kanülenkartusche (112) nach einem der vorhergehenden Ansprüche, wobei das Schutzelement (132) mindestens ein Sicherungselement enthält, um zu verhindern, dass die Nadeleinheit (126) das Schutzelement (132) in einer Richtung von der zweiten Position in die erste Position verlässt.

9. Kit (110), umfassend:
A) mindestens eine Kanülenkartusche (112) nach einem der vorhergehenden Ansprüche; und
B) mindestens eine Einführvorrichtung (116) zum Einführen der Kanüle (114) in ein Körpergewebe (118), wobei die Einführvorrichtung (116) Folgendes umfasst:
- mindestens ein Einführvorrichtungsgehäuse (120) mit mindestens einer Aufnahme (122) zum Aufnehmen der Kanülenkartusche (112); und
- mindestens ein Einführbetätigungselement (124), das konfiguriert ist, um die Nadeleinheit (126) von der ersten Position in die zweite Position und zurück in die erste Position zu treiben.

10. Kit (110) nach dem vorhergehenden Anspruch, wobei das Einführbetätigungselement (124) mindestens ein Treibelement (140) umfasst, das während des Treibens der Nadeleinheit (126) mit dem Eingriffselement (136) des Nadelansatzes (128) verriegelt wird, wobei das Treibelement (140) eine Gabel (142) enthält, die den Nadelansatz (128) während des Treibens der Nadeleinheit (126) von mindestens zwei Seiten umklammert.

11. Kit (110) nach einem der vorhergehenden Ansprüche, die sich auf ein Kit (110) beziehen, wobei das Kit (110) ferner Folgendes umfasst:
C) mindestens einen Pumpenhalter (150), umfassend:
- mindestens eine Befestigungsfläche (152) zur Befestigung an einer Körperoberfläche des Benutzers; und
- mindestens eine Montagefläche (154) gegenüber der Befestigungsfläche (152), wobei die Montagefläche (154) mindestens ein Montageelement (156) zum Montieren entweder der Einführvorrichtung (116) oder einer Medikamentenpumpe an dem Pumpenhalter (150) aufweist.

12. Kit (110) nach dem vorhergehenden Anspruch, wobei das Kit (110) ferner Folgendes umfasst:
D) mindestens eine Medikamentenpumpe, die an dem Pumpenhalter (150) montierbar ist, wobei die Medikamentenpumpe und die Einführvorrichtung (116) austauschbar sind.

13. Verfahren zum Herstellen der Kanülenkartusche (112) nach einem der vorhergehenden Ansprüche, die sich auf eine Kanülenkartusche (112) beziehen, wobei das Verfahren Folgendes umfasst:
i. Bereitstellen der Nadeleinheit (126);
ii. Bereitstellen der Kanüle (114);
iii. Bereitstellen des Schutzelements (132);
iv. Montieren der Kanüle (114) an der Nadel (130) der Nadeleinheit (126);
v. Einführen der Nadeleinheit (126) mit der daran angeordneten Kanüle (114) in das Schutzelement (132) in einer solchen Richtung, dass die Nadel (130) der Nadeleinheit (126) voran eingeführt wird.

## Revendications

1. Cartouche de canule (112) pour recevoir une canule (114), comprenant :
a) une unité d'aiguille (126) ayant un moyeu d'aiguille (128) et une aiguille (130) attachée au moyeu d'aiguille (128) ;
b) une canule (114) ; et
c) un élément protecteur (132),
- dans laquelle l'unité d'aiguille (126) peut être déplacée au sein de l'élément protecteur (132) dans une direction parallèle à l'axe longitudinal de l'aiguille d'au moins une première position à au moins une seconde position et vice versa,
- dans laquelle dans la première position l'aiguille (130) est recouverte par l'élément protecteur (132),
- dans laquelle dans la seconde position l'aiguille (130) s'étend au moins partiellement depuis l'élément protecteur (132),
- dans laquelle dans la première position un élément de rétention (134) de l'élément protecteur (132) vient en prise avec un élément d'accouplement (136) du moyeu d'aiguille (128) par une liaison par complémentarité de forme,
- dans laquelle l'unité d'aiguille (126) est conçue pour être déplacée par un dispositif d'insertion (116) de la première position à la seconde position et de retour à la première position dans laquelle l'élément de rétention (134) vient à nouveau en prise avec l'élément d'accouplement (136) du moyeu d'aiguille (128),
**caractérisée en ce que**
- l'élément de rétention (134) contient au moins un loquet (158),
- le loquet (158) comprend au moins un bras de loquet (160), le bras de loquet (160) étant relié à un boîtier (162) de l'élément protecteur (132) au niveau d'une extrémité fixe (164), le bras de loquet (160) ayant en outre au moins une extrémité libre (166) conçue pour venir en prise avec l'élément d'accouplement (136) du moyeu d'aiguille (128),
- dans laquelle l'extrémité libre (166) contient un élément d'interverrouillage (168) conçu pour venir en prise avec l'élément d'accouplement (136) du moyeu d'aiguille (128),
- dans laquelle l'élément d'interverrouillage (168) et l'élément d'accouplement du moyeu d'aiguille (128) comprennent conjointement au moins deux surfaces inclinées (170, 172), les au moins deux surfaces inclinées (170, 172) comprennent au moins une première surface inclinée (170) mettant en prise de manière coulissante l'élément d'accouplement (136) avec l'élément d'interverrouillage (168) lorsque l'unité d'aiguille (126) se déplace de la première position à la seconde position, et au moins une seconde surface inclinée (172) mettant en prise de manière coulissante l'élément d'accouplement (136) avec l'élément d'interverrouillage (168) lorsque l'unité d'aiguille (126) revient de la seconde position à la première position, de telle sorte qu'en déplaçant l'unité d'aiguille (126) de la première position à la seconde position la liaison par complémentarité de forme est déverrouillée et en déplaçant l'unité d'aiguille (126) de la seconde position à la première position la liaison par complémentarité de forme est verrouillée.

2. Cartouche de canule (112) selon la revendication précédente, dans laquelle l'élément d'interverrouillage (168) comprend les au moins deux surfaces inclinées (170, 172), l'au moins une première surface inclinée (170) interagissant avec au moins un bord (174) de l'élément d'accouplement (136) lorsque l'unité d'aiguille (126) se déplace de la première position à la seconde position, et l'au moins une seconde surface inclinée (172) interagissant avec au moins un bord proximal (176) du moyeu d'aiguille, lorsque l'unité d'aiguille revient de la seconde position à la première position, de telle sorte qu'en déplaçant l'unité d'aiguille (126) de la première position à la seconde position la liaison par complémentarité de forme est déverrouillée et en déplaçant l'unité d'aiguille (126) de la seconde position à la première position la liaison par complémentarité de forme est verrouillée.

3. Cartouche de canule (112) selon l'une quelconque des revendications précédentes, dans laquelle l'élément d'accouplement (136) comprend une rainure circonférentielle (144) dans le moyeu d'aiguille (128).

4. Cartouche de canule (112) selon l'une quelconque des revendications précédentes, dans laquelle le loquet (158) est conçu pour venir en prise avec au moins une cavité dans au moins une surface de l'élément d'accouplement (136).

5. Cartouche de canule (112) selon l'une quelconque des revendications précédentes, dans laquelle les au moins deux surfaces inclinées (170, 172) ont des pentes différentes.

6. Cartouche de canule (112) selon l'une quelconque des revendications précédentes, dans laquelle le loquet (158), lorsque l'élément de rétention (134) est en prise avec l'élément d'accouplement (136) du moyeu d'aiguille (128), est dans un état relâché.

7. Cartouche de canule (112) selon l'une quelconque des revendications précédentes, dans laquelle l'élément protecteur (132) a un logement (162) avec un alésage contenu à l'intérieur de celui-ci, dans laquelle l'unité d'aiguille (126) est reçue au sein de l'alésage d'une manière pouvant coulisser de manière linéaire dans une direction parallèle à l'axe (138) de l'aiguille (130).

8. Cartouche de canule (112) selon l'une quelconque des revendications précédentes, dans laquelle l'élément protecteur (132) contient au moins un élément de fixation pour empêcher l'unité d'aiguille (126) de quitter l'élément protecteur (132) dans une direction de la seconde position à la première position.

9. Kit (110) comprenant :
A) au moins une cartouche de canule (112) selon l'une quelconque des revendications précédentes ; et
B) au moins un dispositif d'insertion (116) pour insérer la canule (114) dans un tissu corporel (118), le dispositif d'insertion (116) comprenant :
- au moins un logement de dispositif d'insertion (120) ayant au moins un réceptacle (122) pour recevoir la cartouche de canule (112) ; et
- au moins un actionneur d'insertion (124) conçu pour entraîner l'unité d'aiguille (126) de la première position à la seconde position et de retour à la première position.

10. Kit (110) selon la revendication précédente, dans lequel l'actionneur d'insertion (124) comprend au moins un élément d'entraînement (140) s'interverrouillant avec l'élément d'accouplement (136) du moyeu d'aiguille (128) pendant l'entraînement de l'unité d'aiguille (126), dans lequel l'élément d'entraînement (140) contient une fourche (142) enserrant le moyeu d'aiguille (128) sur au moins deux côtés pendant l'entraînement de l'unité d'aiguille (126).

11. Kit (110) selon l'une quelconque des revendications précédentes se référant à un kit (110), dans lequel le kit (110) comprend en outre :
C) au moins un support de pompe (150) comprenant :
- au moins une surface d'attachement (152) pour l'attachement à une surface corporelle de l'utilisateur ; et
- au moins une surface de montage (154) opposée à la surface d'attachement (152), la surface de montage (154) ayant au moins un élément de montage (156) pour monter soit le dispositif d'insertion (116) soit une pompe à médicament sur le support de pompe (150).

12. Kit (110) selon la revendication précédente, le kit (110) comprenant en outre :
D) au moins une pompe à médicament pouvant être montée sur le support de pompe (150), dans lequel la pompe à médicament et le dispositif d'insertion (116) sont interchangeables.

13. Procédé de fabrication de la cartouche de canule (112) selon l'une quelconque des revendications précédentes se référant à une cartouche de canule (112), le procédé comprenant :
i. la fourniture de l'unité d'aiguille (126) ;
ii. la fourniture de la canule (114) ;
iii. la fourniture de l'élément protecteur (132) ;
iv. le montage de la canule (114) sur l'aiguille (130) de l'unité d'aiguille (126) ;
v. l'insertion de l'unité d'aiguille (126) avec la canule (114) disposée sur celle-ci dans l'élément protecteur (132), dans une direction telle que l'aiguille (130) de l'unité d'aiguille (126) est insérée devant.
